# EUROPÄISCHE PATENTSCHRIFT

(11) **EP 3 066 186 B1**
(45) Veröffentlichungstag und Bekanntmachung des Hinweises auf die Patenterteilung: **11.10.2017**
(21) Anmeldenummer: 14814739.0
(22) Anmeldetag: 03.11.2014
(51) Int. Cl.: C12M 1/00, C12M 3/00, C12M 1/09

(54) **PHOTOBIOREAKTOR UND PHOTOBIOREAKTORFARM**
PHOTOBIOREACTOR AND PHOTOBIOREACTOR FARM
PHOTOBIORÉACTEUR ET PARC DE PHOTOBIORÉACTEURS

(30) Priorität: 08.11.2013 DE 102013112291
(43) Veröffentlichungstag der Anmeldung: 14.09.2016
(73) Patentinhaber: Phytolutions GmbH, 28759 Bremen (DE)
(72) Erfinder: THOMSEN, Claudia, 28759 Bremen (DE)
(74) Vertreter: Weidner Stern Jeschke
(86) Internationale Anmeldenummer: PCT/DE2014/100389
(87) Internationale Veröffentlichungsnummer: WO 2015/067241

(56) Entgegenhaltungen:
- EP-A1- 2 360 235
- WO-A1-2011/098076
- WO-A2-2009/090549
- WO-A2-2009/094196
- DE-A1-102007 000 815
- GB-A- 2 471 492
- US-A1- 2011 287 541
- US-A1- 2012 231 528

## Beschreibung

Die Erfindung betrifft einen Photobioreaktor zum Kultivieren von Algen, welcher wenigstens zwei Kultivierungskammern aufweist, welche durch eine erste Verbindung miteinander verbunden sind. Des Weiteren betrifft die Erfindung eine Photobioreaktorfarm.

Autotrophe Algen und Prokaryonten werden in sogenannten Photobioreaktoren kultiviert und auch in großen Mengen produziert. Üblicherweise kann die Kultivierung neben der Autotrophie auch durch Mixotrophie und/oder Heterotrophie erfolgen.

Grundsätzlich wird bei Photobioreaktoren zwischen starren Röhrensystemen beispielsweise aus Glas, Polykarbonat oder Plexiglas sowie flexiblen Foliensystemen, wie beispielsweise aus Polyethylen oder anderen Folienmaterialien, unterschieden.

Häufig werden die Foliensysteme vertikal als Beutel, flächig als dünne Mehrkammerbeutel oder auch starr als sogenannte Flat-Panel-Bioreaktoren eingesetzt.

Für die Auslegung von Photobioreaktoren und für den Anwender wichtige Produktionsparameter sind eine möglichst große photosynthetische, aktive Oberfläche des Photobioreaktors, bevorzugt in Verbindung mit einem geringen Lichtweg, sodass eine relative und auch absolute hohe Produktivität gewährleistet werden kann. Weitere Anforderungen an einen Photobioreaktor liegen in seiner Skalierbarkeit zu einem großindustriellen Maßstab, im Preis-LeistungsVerhältnis, der universellen Einsetzbarkeit und der mechanischen Belastbarkeit.

Photobioreaktoren werden in landseitigen Produktionsstätten und in sogenannten Algenfarmen in natürlichen Gewässern eingesetzt. Bei Algenfarmen sind beispielsweise eine Flächen von 60km² für die Produktion von Makroalgen bekannt.

Beim Einsatz eines Photobioreaktors in natürlichen Gewässern (Fluss, See, Meer) treten weitere Einflussfaktoren auf. Der Wind und die Strömungs- und Wellenbewegungen können sich negativ auswirken, wenn beispielsweise das Material des Photobioreaktors der mechanischen Belastung nicht standhält. Sofern nicht jeder Photobioreaktor einzeln aufgestellt ist, werden beispielsweise parallel aufgehängte Photobioreaktoren durch die Strömung aneinander getrieben und beschatten sich dadurch gegenseitig. Zudem wirken sich natürliche Algenblüten, insbesondere sogenannte Algenteppiche auf dem Meer nachteilig aus, da diese ebenfalls zu einer Beschattung von Photobioreaktoren führen können.

Aus der AU 2009205386 B2 ist ein Photobioreaktor bekannt, welcher zur Verminderung des Problems der Agglomeration innerhalb der Kultivierungskammern in mehrere Subkompartimente unterteilt ist und bei dem die Position des Photobioreaktors im Wasser über Dichtedifferenzen zwischen dem Kultivierungsmedium und dem umgebenen Wasser eingestellt wird.

In der WO 2009/094196 A2 ist ein Photobioreaktor zum Kultivieren von Algen offenbart, welcher durch Auftriebsmittel tauchfähig ausgestaltet ist.

Zudem sind in der DE 10 2007 000 815 A1 Freiluftbioreaktoren mit artifiziellen Strahlungsquellen offenbart, wobei als Strahlungsquellen ein oder mehrere aus der Gruppe, enthaltend mit LEDs bestückte Formteile, Lichtleiter, lumineszierende Formteile eingesetzt werden, welche im Inneren des Reaktors angeordnet sind.

Nachteilig ist beim Stand der Technik, dass mehrere parallel gesetzte Photobioreaktoren sich unter Einfluss der Strömung- und Wellenbewegung insbesondere horizontal und auch vertikal neigen können, sodass sie aneinander getrieben werden und sich gegenseitig beschatten, sodass die Produktivität vermindert wird.

Insbesondere bei starken Strömung- und Wellenbewegung besteht zudem die Gefahr, dass das Photobioreaktormaterial versagt, insbesondere dass Foliensysteme einreißen. Dadurch können die Kultivierungskammern geöffnet und die Algen ins umgebende Wasser freigesetzt werden oder bei Abriss am Befestigungssystem kann der gesamte Photobioreaktor im Meer verloren gehen.

Aufgabe der Erfindung ist es, den Stand der Technik zu verbessern.

Gelöst wird die Aufgabe durch einen Photobioreaktor zum Kultivieren von Algen, welcher wenigstens zwei Kultivierungskammern aufweist, welche durch eine erste Verbindung miteinander verbunden sind, wobei die erste Verbindung eine Sollbruchstelle aufweist, welche sich bei einer Grenzströmung öffnet, sodass ein strömendes Medium, insbesondere Meerwasser, durch die geöffnete Sollbruchstelle hindurch fließt und somit einen Strömungswiderstand des Photobioreaktors vermindert wird.

Dadurch passt sich die Form der Kultivierungskammer des Photobioreaktors den Strömungs- und Wellenbedingungen des Einsatzgebietes an. Bei zu starker Strömung öffnet sich die Sollbruchstelle, sodass Wasser durch die Sollbruchstelle und um die Kultivierungskammer herum strömen kann, wodurch der Strömungswiderstand und Wellenwiderstand des Photobioreaktors vermindert wird. Zudem können sich die Kultivierungskammern dadurch flexibler entsprechend der Strömung ausrichten. Somit wird verhindert, dass der Photobioreaktor aufgrund zu starker Strömung zerstört wird, zum Beispiel bei einem Folienreaktor die Folie einreißt, oder vom Befestigungssystem aufgrund zu starker Strömungsbelastung abreißt.

Folglich ist durch diese Lösung eine höhere Produktivität und Stabilität des Photobioreaktors realisierbar.

Ein wesentlicher Gedanke der Erfindung beruht darauf, dass entgegen dem Stand der Technik die Kultivierungskammern eines Photobioreaktors nicht beständig fest verbunden sind, sondern dass die Verbindung eine Sollbruchstelle aufweist, welche sich bei einer Strömung mit starken Scherkräften öffnet, sodass das Wasser durch die geöffnete Sollbruchstelle hindurch und um die Kultivierungskammern herum fließen kann. Somit sind die Scherkräfte auf den Photobioreaktor vermindert.

Folgendes Begriffliche sei erläutert:

Bei einem "Photobioreaktor" handelt es sich insbesondere um einen Bioreaktor zur Anzucht und Produktion von Mikroorganismen und/oder Makroorganismen, insbesondere Algen, außerhalb ihrer natürlichen und innerhalb einer künstlichen technischen Umgebung. Der Photobioreaktor weist insbesondere Kultivierungskammern auf, in denen die Organismen von der Umgebung abgetrennt kultiviert werden. Ein Photobioreaktor dient insbesondere zur Kultivierung von phototrophen Organismen, welche natürliches und/oder künstliches Licht zur eigenen Energiegewinnung nutzen. In einem Photobioreaktor werden insbesondere Organismen kultiviert, welche den Prozess der Photosynthese nutzen, um aus Licht und CO₂ ihre eigene Biomasse aufzubauen. Somit dient ein Photobioreaktor insbesondere zur kontrollierten Bereitstellung eines Lebensraumes, welcher für den oder die relevanten Organismen möglichst die optimalen Lebensbedingungen bietet. Ein Photobioreaktor ermöglicht insbesondere deutlich höhere Wachstums- und Produktionsraten sowie Reinheiten der Organismen, als dies in natürlichen Gewässern erzielbar ist.

Unter "Kultivieren" wird insbesondere die Schaffung und Aufrechterhaltung von Bedingungen verstanden, welche ein Wachstum von bestimmten Organismen gewährleisten. Mit "Kultivieren" ist insbesondere auch die Vermehrung von Organismen verbunden.

"Algen" sind insbesondere im Wasser lebende, eukaryotische, pflanzenartige Organismen, welche Photosynthese betreiben und/oder Protisten, also ein- bis wenigzellige Eukaryoten wie Grün- und Rotalgen, welche autotroph aber auch heterotroph, mixotroph, aerobe oder anaerob leben können. Zudem werden unter Algen insbesondere auch die Cyanobakterien (auch Blaualgen genannt) verstanden, welche als Bakterien zu den Prokaryoten zählen. Die Cyanobakterien betreiben insbesondere oxygene oder anoxygene Photosynthese, sodass teilweise keine CO₂-Fixierung in der Biomasse stattfindet. Unter Algen wird insbesondere auch das natürlich vorkommende pflanzliche assimilatorisch aktive Plankton im Meer verstanden. Algen können insbesondere eine Größe von < 0,2µm (Femtoplankton), < 2µm (Picoplankton) bis > 2mm (Makroplankton) und Prokaryoten insbesondere eine Größe von 0,2µm bis 700µm annehmen.

Eine "Verbindung" bezeichnet insbesondere einen mechanischen Zusammenhalt zwischen zwei Kultivierungskammern. Dabei handelt es sich insbesondere um eine feste Verbindung und/oder eine Verbindung, welche die Beweglichkeit zwischen den beiden Kultivierungskammern einschränkt. Die Verbindung ist insbesondere stoffschlüssig. Zum Beispiel sind bei einem Endlosfolienreaktor die Verbindungen der Kultivierungskammern durch Schweißen gebildet worden und das Folienmaterial verbindet die beiden Kultivierungskammern zwischen den Schweißstellen.

Unter einer "Sollbruchstelle" wird insbesondere ein gezielt vorgesehenes Konstruktionselement verstanden, welches im Schadens- und/oder Überlastungsfall gezielt und vorhersagbar versagt. Insbesondere öffnet sich eine Sollbruchstelle bei einer äußeren Grenzströmung des Wassers, sodass das Wasser durch die geöffnete Sollbruchstelle hindurch- und um die Kultivierungskammern herum fließt. Somit hat die Sollbruchstelle insbesondere die Aufgabe, eine Durchströmung des Photobioreaktors zu ermöglichen und den Strömungswiderstand des Photobioreaktors bei einer hohen Scherkraft der Strömung zu vermindern. Trotz der geöffneten Sollbruchstelle bleibt insbesondere die Verbindung zwischen den Kultivierungskammern bestehen.

Unter einem "strömenden Medium" wird insbesondere die sich bewegende Luft und/oder das sich bewegende Wasser verstanden, welche oder welches auf dem Photobioreaktor mit einer Strömungsgeschwindigkeit auftrifft. Insbesondere kann es sich bei dem strömenden Medium um Meerwasser handeln, jedoch ist es auch möglich, dass der Photobioreaktor auf Gestellen oberhalb des Meeresspiegels angeordnet ist, um eine intensive Bestrahlung zu gewährleisten. In diesem Fall ist insbesondere Luft das strömende Medium.

Unter "Grenzströmung" wird insbesondere die Strömung eines strömenden Mediums verstanden, welche eine derartige Scherkraft auf den Photobioreaktor ausübt, dass der Photobioreaktor sich verformt und/oder von seiner vertikalen und/oder horizontalen Position im Gewässer abweicht, welche der Photobioreaktor vor Auftreten der Grenzströmung eingenommen hat. Die Grenzströmung kann insbesondere beim Übergang von einer laminaren zu einer turbulenten Strömung vorliegen. Jedoch kann die Grenzströmung insbesondere auch unter anderen Strömungsbedingungen eintreten, da die Strömungs- und Wellenbewegungen sowie der Wind zusammenwirken können, sodass lokal am Photobioreaktor unterschiedliche Belastungsverhältnisse vorliegen können. Zudem besteht insbesondere auch eine Abhängigkeit von der Fläche des Photobioreaktors.

Ein "Strömungswiderstand" ist insbesondere ein Resultat der Kraft, welche die Strömung auf den Photobioreaktor ausübt. Auf die Oberfläche des umströmten Photobioreaktors wirkt die Strömung örtlich unterschiedlich Schubspannung und Druck aus. Die resultierende Kraft lässt sich durch Aufintegration von Druck und Schubspannungen über die gesamte Oberfläche bestimmen. Hierbei ist die Widerstandskraft die Kraftkomponente, welche in Richtung der Anströmrichtung vorliegt.

In einer weiteren Ausführungsform des Photobioreaktors ist die Sollbruchstelle durch eine Begrenzungsstruktur, insbesondere eine verstärkte Begrenzungsnaht, in einer Sollbruchausdehnung begrenzt.

Durch die Begrenzungsstruktur kann die Sollbruchstelle sich maximal nur soweit öffnen, wie dies konstruktiv vorgesehen ist. Des Weiteren wird durch die Begrenzungsstruktur sichergestellt, dass die Sollbruchstelle bei hohen Strömungsbelastungen sich nicht weiter öffnet oder sogar einreißt. Dies kann insbesondere durch eine verstärkte Begrenzungsstruktur sichergestellt werden.

Somit kann beispielsweise eine verstärkte Begrenzungsnaht eingesetzt werden, wobei es sich um eine Schweißnaht und/oder auch um eine genähte Naht handeln kann, welche durch Anzahl der Schweißungen und/oder der Nähte gezielt verstärkt und an die Strömungs- und Wellenbelastung des Einsatzgebietes angepasst werden kann.

Zudem wird über die Begrenzungsstruktur und deren Stärke festgelegt, wie weit sich die Kultivierungskammern beim Auftreffen einer Grenzströmung verformen können.

Eine "Begrenzungsstruktur" ist insbesondere eine Struktur, welche derart ausgelegt ist, dass die Ausdehnung der Sollbruchstelle am Anfang und/oder am Ende der Sollbruchstelle begrenzt ist und insbesondere nicht über die konstruktiv vorgesehene Ausdehnung hinaus eine Öffnung der Sollbruchstelle ermöglicht wird. Die Begrenzungsstruktur ist insbesondere eine verstärkte Struktur am Material des Photobioreaktors, um die Sollbruchausdehnung zu begrenzen. Die Begrenzungsstruktur ist insbesondere eine verstärkte Begrenzungsnaht, zum Beispiel eine verstärkte Schweißnaht und/oder eine konventionelle Naht.

Unter "Sollbruchausdehnung" wird insbesondere die konstruktiv vorgesehene Ausdehnung der Sollbruchstelle als maximale Abmessung verstanden.

Um eine zuverlässige Öffnung der Sollbruchstelle zu gewährleisten, ist die Sollbruchstelle des Photobioreaktors eine Sollbruchnaht.

Durch die neu entwickelte Schweißnaht ist es möglich, dass beispielsweise vertikal verlaufene Schweißnähte bei zu hoher Belastung entlang einer Sollbruchnaht aufreißen, wobei die Sollbruchnaht insbesondere durch Perforation des Folienmaterials zwischen den Schweißnähten, welche die Kultivierungskammern begrenzen, ausgebildet werden kann. Dadurch reißt der Photobioreaktor beim Überschreiten einer bestimmten Strömung oder durch Wellenbewegung erzeugte Scherkraft entlang der Perforation auf und erlaubt ein Durchströmen der Folien durch den entstandenen Spalt zwischen den beiden Kultivierungskammern. Die dadurch bewirkte Verminderung der Angriffsfläche führt zu einer signifikanten Entlastung des Photobioreaktors.

Sobald die Strömung wieder abnimmt, verkleinert sich der Spalt zwischen den beiden Kultivierungskammern und der Photobioreaktor kann seine ursprüngliche Form wieder annehmen.

Durch die Sollbruchstelle können sich die Kultivierungskammern insbesondere in horizontaler und/oder vertikaler Richtung verformen. Durch die Änderung der Form der Kultivierungskammern kann sich entsprechend auch ihr Auftrieb verändern.

Unter einer "Sollbruchnaht" wird insbesondere eine konstruktiv vorgesehene Naht verstanden, welche bei Einwirken einer Grenzströmung dazu führt, dass die Sollbruchstelle sich öffnet. Eine Sollbruchnaht ist insbesondere eine Perforation zwischen den begrenzenden Schweißnähten zweier Kultivierungskammern und/oder eine konventionelle Naht, wobei die durch das Setzen der Naht entstandenen Löcher zum Öffnen der Sollbruchstelle führen.

In einer weiteren Ausführung des Photobioreaktors ist die Sollbruchstelle ein Wiederverschluss, insbesondere ein Klettverschluss.

Durch die Anzahl und Position der Widerhäkchen im Wiederverschluss kann gezielt eine Anpassung an die Grenzströmung erfolgen, bei der eine Öffnung der Sollbruchstelle auftreten soll, sowie an das Einsatzgebiet des Photobioreaktors.

Vorteilhaft können sich die flexiblen Widerhäkchen des Wiederverschlusses bei Annäherung in Folge der Unterschreitung der Grenzströmung wieder verbinden. Auch kann der Wiederverschluss manuell wieder durch Druck verschlossen werden, wobei dies direkt im Einsatzgebiet oder bei Entnahme des Photobioreaktor aus dem Wasser zum Beispiel zu Wartungszwecken oder bei Änderung des Einsatzgebietes erfolgen kann.

Ein "Wiederverschluss" ist insbesondere ein Verschluss, der reversibel geöffnet und somit wieder geschlossen werden kann. Insbesondere öffnet sich der Wiederverschluss selbsttätig bei Anliegen einer bestimmten Grenzströmung und schließt sich bei Unterschreiten der Grenzströmung wieder. Bei dem Wiederverschluss kann es sich insbesondere um einen Klettverschluss handeln, welcher insbesondere überwiegend ein textiles, beliebig oft zu lösendes Verschlussmittel auf dem Prinzip von Klettfrüchten aufweist. Ein Klettverschluss besteht insbesondere aus zwei gewählten Chemiefaserstreifen, wovon einer flexible Widerhäkchen und der andere Schlaufen aufweist. Insbesondere im zusammengepressten Zustand ergibt sich ein belastungsfähiger aber reversibler Schnellverschluss. Gewebte Klettbänder können insbesondere aus Polyamid-, Polyester- und Polyolefinfasern und/oder Polyaramiden bestehen. Der Klettverschluss ist insbesondere an das Material des Photobioreaktors verschweißt und/oder genäht und/oder verklebt.

Um eine Umströmung und Verformung der Kultivierungskammern des Photobioreaktor zu gewährleisten und die Stabilität des Photobioreaktors insgesamt nicht zu gefährden, weist die Sollbruchstelle eine maximale Sollbruchausdehnung von 5% bis 95% einer vertikalen und/oder horizontalen und/oder schrägen äußeren Abmessung des Photobioreaktors auf.

Des Weiteren wird dadurch gewährleistet, dass ausreichend Platz am Material des Photobioreaktors verbleibt, um Ver- und Entsorgungseinrichtungen und/oder Befestigungseinrichtungen und/oder Ankersysteme und/oder Ballastsysteme am Photobioreaktor anzuordnen.

Da der Photobioreaktor in einer dynamischen Umgebung, insbesondere mit hohen Wellenbewegungen, eingesetzt werden kann, ist unter einer vertikalen und/oder horizontalen und/oder schrägen Abmessung des Photobioreaktors die Ausrichtung im Verhältnis zur gemittelten Wasseroberfläche zu verstehen.

In einer weiteren Ausführungsform des Photobioreaktors weist der Photobioreaktor weitere Kultivierungskammern auf, welche jeweils mit einer weiteren Verbindung an eine angrenzende Kultivierungskammer angeordnet sind.

Ausgehend von einem Endlosfolienschlauch von üblicherweise 1,0m bis 10,0m Umfang, kann durch das gezielte Setzen von Schweißnähten ein Photobioreaktor mit mehreren Kultivierungskammern produziert werden, wobei die Kultivierungskammern jeweils mit einer weiteren Verbindung an eine angrenzende Kultivierungskammer angeordnet sind.

Für eine optimale Kultivierung und eine optimale Belastung des Photobioreaktors haben die begrenzenden Schweißnähte der Kultivierungskammern insbesondere einen Abstand von 5cm bis 50cm, bevorzugt von 10cm bis 20cm.

Bei Höhen von 0,5m bis 2,0m können dadurch Photobioreaktoren mit Längen von insbesondere 10m, aber auch bis zu 5.000m produziert werden.

Durch die Anordnung von mehreren Kultivierungskammern kann eine möglichst große photosynthetische, aktive Oberfläche für die Produktion geschaffen werden. Somit kann die Produktion auch bis in den großindustriellen Maßstab skaliert und ein günstiges Preis/Leistungsverhältnis erzielt werden.

Damit bei einem Photobioreaktor mit mehr als zwei angeordneten Kultivierungskammern ein Öffnen der weiteren Verbindungen ermöglicht wird, weisen die weiteren Verbindungen weitere Sollbruchstellen auf.

Insbesondere bei einem Photobioreaktor mit einer Vielzahl von Kultivierungskammern und somit entsprechendem Strömungswiderstand, sind Sollbruchstellen an den weiteren Verbindungen vorteilhaft, um ein Öffnen und Durchströmen der weiteren Sollbruchstellen zu ermöglichen. Dadurch wird die Strömungsbelastung ebenfalls auf großflächige Photobioreaktoren vermindert. Somit ist auch eine Verformbarkeit von sehr langen und/oder großflächigen Photobioreaktoren gewährleistet.

In einer weiteren Ausführungsform des Photobioreaktors ist eine der Sollbruchstellen oder sind mehrere Sollbruchstellen vertikal und/oder horizontal und/oder schräg, insbesondere parallel zueinander, ausgestaltet.

Durch diese Ausrichtung der Sollbruchstellen kann die Strömungsbelastung gleichmäßig vom Photobioreaktor genommen werden, indem die Sollbruchstellen simultan öffnen.

Insbesondere ist es vorteilhaft, wenn die Sollbruchstellen vertikal und/oder horizontal und/oder schräg, jedoch im gleichen Abstand parallel zueinander ausgestaltet sind, da damit gleichmäßig die Belastung vom Photobioreaktor gemindert wird.

Zudem ist bei gleichzeitiger Begasung des Photobioreaktors eine vertikale Ausrichtung der Kultivierungskammern und der Sollbruchstellen insbesondere dann zu bevorzugen, wenn aufgrund der Algenart und/oder des Kultivierungsmediums oder anderer Einflussgrößen mit einer Sedimentation oder selektiven Anreicherung der Algen und/oder des Kultivierungsmediums in den Kultivierungskammern zu rechnen ist. Durch die vertikale Ausrichtung und die aufsteigenden Gasblasen wird eine Sedimentation verhindert und eine homogene Vermischung gefördert. Dies ist insbesondere vorteilhaft, wenn im Gewässer keine oder nur geringe die Durchmischung fördernde Bedingungen wie starke Wellenbewegung und Strömung vorliegen.

Wie bereits oben erläutert, bezieht sich die vertikale und/oder horizontale und/oder schräge Ausrichtung der Sollbruchstellen auf die gemittelte Wasseroberfläche.

Um eine Photosynthese der Algen zu ermöglichen, weisen die Kultivierungskammern ein lichtdurchlässiges Material auf, insbesondere ein Folienmaterial aus Polyethylen.

Hierbei kann das natürliche und/oder künstliche Licht durch das lichtdurchlässige Material vollständig und/oder selektiv nur bestimmte Lichtanteile durchgelassen werden.

In Einsatzgebieten mit starker Lichtintensität kann es insbesondere ein Vorteil sein, ein lichtdurchlässiges Material zu verwenden, welches gleichzeitig die natürliche Lichtintensität abschwächt, um einen Stress für die Algen zu vermeiden.

Ebenso kann das lichtdurchlässige Material zusätzlich eine Wärmeisolationsfunktion aufweisen, um eine zu starke Erwärmung und/oder Abkühlung in den Kultivierungskammern zu vermeiden.

Unter "Folienmaterial" wird insbesondere eine Kunststofffolie verstanden, welche ein sehr dünnes (beispielsweise < 1 mm) Blatt aus Kunststoff darstellt. Das Folienmaterial wird insbesondere in einer Endlosbahn gefertigt, vorgeschweißt sowie die Sollbruchstellen angebracht und anschließend aufgerollt. Je nach gewünschter Länge des Photobioreaktors wird die vorgefertigte Endlosbahn abgeschnitten und beide Schnittseiten verschweißt, sodass der Photobioreaktor später abgeschlossen gegenüber dem Gewässer eingesetzt werden kann. Als lichtdurchlässiges Folienmaterial wird insbesondere Polyethylen eingesetzt, aber es können auch andere Folienmaterialien mit lichtdurchlässigen Eigenschaften, wie zum Beispiel Polyamid, PVC oder Ethylen-Tetrafluorethylen verwendet werden.

In einer weiteren Ausführungsform des Photobioreaktors weisen die Kultivierungskammern Meerwasser als Kultivierungsmedium und/oder wenigstens ein Mehrwegeventil zum Gasaustausch mit dem umgebenden Medium, insbesondere Luft oder Meerwasser, und/oder ein Versorgungsschlauch auf, welcher derart ausgestaltet ist, dass die Kultivierungskammer mit Nährstoffen, insbesondere Gasen und/oder angereichertem Wasser versorgt werden.

Dadurch, dass die Kultivierungskammer Meerwasser als Kultivierungsmedium aufweisen, kann direkt das umgebende Meerwasser als Kultivierungsmedium verwendet werden und ein Kultivierungsmedium muss nicht aufwendig künstlich hergestellt werden.

Ein Mehrwegeventil zum Gasaustausch mit dem umgebendem Medium ist zum einem vorteilhaft, da der Photobioreaktor üblicherweise mit CO₂ und/oder Luft begast wird, um die Produktion zu erhöhen und um die Nährstoffe und die Algen homogen im Reaktor zu verteilen. Überschüssige Gase treten über das Mehrwegeventil aus dem Photobioreaktor aus.

Zum anderen kann das Mehrwegeventil auch zur Regulierung der Position des Photobioreaktors in der vertikalen Wassersäule dienen, da sich darüber der Gasinhalt und somit der Auftrieb des Photobioreaktors einstellen lässt. Insbesondere bei hohem Wellengang und sehr starker Strömung ist es vorteilhaft den Auftrieb des Photobioreaktors über das Mehrwegeventil zu vermindern, sodass der Photobioreaktor unter die Meeresoberfläche absinkt und eine tiefere Position in der vertikalen Wassersäule einnimmt.

Diese Auftriebseinstellung ist insbesondere vorteilhaft, wenn aufgrund sehr hoher Strömungsbelastung und/oder hohem Wellengang die Öffnung der Sollbruchstellen nicht mehr ausreichen, um eine Beschädigung des Photobioreaktors zu verhindern.

Zudem kann durch die Auftriebseinstellung über das Mehrwegeventil der Photobioreaktor in der vertikalen Wassersäule so positioniert werden, dass optimale Wachstumsbedingungen, insbesondere optimale Lichtverhältnisse, vorliegen.

Ein "Versorgungsschlauch", welcher die Kultivierungskammern mit Nährstoffen, insbesondere mit Gasen und/oder angereichertem Wasser, versorgt, dient einer optimalen Produktion der Algen. Insbesondere ist es vorteilhaft, dass der Versorgungsschlauch am Boden des Photobioreaktors angeordnet ist, da bei Begasung feinperlig erzeugte Gasblasen im Medium absorbiert werden und zusätzlich zur Erzeugung eines erforderlichen Auftriebs zum oberen Teil des Photobioreaktors aufsteigen. In diesem Fall sind die Kultivierungskammern und die Verbindungen vertikal zur gemittelten Wasseroberfläche ausgerichtet. Insbesondere kann der Versorgungsschlauch auch in der Tiefe und/oder Höhe verstellt werden. Der Versorgungsschlauch kann insbesondere auch als Ernteschlauch ausgebildet sein.

Auch ist es möglich, durch Verminderung oder Abschalten der Gaszufuhr über den Versorgungsschlauch den Auftrieb des Photobioreaktors zu mindern und dadurch diesen unter die Meeresoberfläche abzusenken.

Ein "Kultivierungsmedium" (auch "Nährmedium" genannt) dient insbesondere zur Versorgung und Kultivierung von Organismen insbesondere Algen. Als Kultivierungsmedium wird insbesondere eine flüssige Nährlösung eingesetzt, welche bevorzugt aus Wasser, insbesondere Meerwasser, mindestens eine verwertbare Energiequelle wie organische oder schwefelhaltige Verbindungen, benötigte Nährstoffe (organische und/oder anorganische Kohlenstoff-, Stickstoff, Schwefel- und/oder Phosphatquellen sowie andere essentielle Nährstoffe) und Spurenelemente besteht.

Um den Photobioreaktor im Wasser auszurichten, weist der Photobioreaktor ein Beschwerungselement und/oder ein Verankerungselement auf, wodurch dem Photobioreaktor eine Ausrichtungsrichtung aufprägbar ist.

Durch ein Beschwerungselement und/oder ein Verankerungselement kann insbesondere eine Ausrichtung des Photobioreaktors entgegen dem wirkenden Auftrieb erfolgen, sodass der Photobioreaktor in der Schwebe gehalten, weiter zum Meeresboden absinken und/oder am Meeresboden befestigt werden kann.

Dies ist insbesondere dann vorteilhaft, wenn die Sollbruchnähte bei sehr starker Strömung nicht mehr ausreichen und/oder eine Verminderung des Auftriebes über das Mehrwegeventil und die Begasung nicht weiter möglich ist, jedoch der Photobioreaktor aufgrund der Belastung aber tiefer in Meeresbodennähe gebracht werden soll.

Ein Absenken in Richtung des Meeresbodens kann auch vorteilhaft bei einer heterotrophen Produktion sein, welche kein Licht benötigt, und/oder einer mixotrophen Produktion.

Des Weiteren kann im Falle der Produktionssteigerung bei erhöhter Begasung der dadurch entstandene Auftrieb durch ein Beschwerungs- und/oder Verankerungselement ausgeglichen und der Photobioreaktor in Schwebe gehalten werden.

Ein "Beschwerungselement" ist insbesondere ein Element, welches eine Dichte aufweist, die schwerer ist als das Kultivierungsmedium und/oder das umgebene Wasser. Bei dem Beschwerungselement kann es sich insbesondere um eine Flüssigkeit und/oder einen Feststoff handeln. Beispielsweise kann es sich um eine Sand- und/oder Wasserballast handeln. Das Beschwerungselement kann insbesondere in einer speziellen, durch eine Naht abgetrennten Kammer ausgeführt sein.

Ein "Verankerungselement" stellt insbesondere eine zugsichere Verbindung zwischen dem Photobioreaktor und dem Meeresboden her. Dabei nimmt das Verankerungselement insbesondere Zugspannung auf. Das Verankerungselement kann beispielsweise aus Holz und/oder Eisen und/oder Stahl gefertigt sein. Das Verankerungselement kann insbesondere auch in Verbindung mit bestehenden oder geplanten Offshore-Anlagen wie Windenergieanlagen ausgeführt sein, um Synergie beispielsweise in der Verankerung, der Wärme-/ Abwärmenutzung, bei der Versorgung und Wartung zu nutzen.

Um einen Auftrieb des Photobioreaktors zu ermöglichen, ist der Photobioreaktor derart einrichtbar, dass durch ein Ent- und/oder Begasen und/oder Verdünnen und/oder Aufkonzentrieren eines in den Kultivierungskammern befindlichen Kultivierungsmediums ein Auftrieb des Photobioreaktors einstellbar ist.

Dadurch kann die Position des Photobioreaktors in der vertikalen Wassersäule weiter eingestellt werden. Bei Schwachlicht oder gewünschtem Lichtstress kann dadurch der Photobioreaktor direkt an die Wasseroberfläche gebracht werden. Bei zu starker Lichtintensität kann der Photobioreaktor dagegen unterhalb der Wasseroberfläche positioniert werden. Damit ist es möglich, die Position des Photobioreaktors in der vertikalen Wassersäule zu optimieren und beispielsweise den Tagesverlauf des Lichtes sowie den jahreszeitlichen Erfordernissen und den Wellen- und Windverhältnissen anzupassen.

Im einen zusätzlichen Aspekt der Erfindung wird die Aufgabe gelöst durch eine Photobioreaktorfarm, welche einen Photobioreaktor wie zuvor beschrieben aufweist.

Durch eine Photobioreaktorfarm kann auf minimalem Raum eine sehr große photosyntheseaktive Fläche bereitgestellt werden.

Photobioreaktorsegmente, beispielsweise mit einer Länge von 2m bis 12m, bevorzugt 3m bis 10m können mittels Befestigungsmitteln wie Kunststoffringen, Rollen, Karabinerhaken und/oder ähnlichen Vorrichtungen zum Beispiel an gespannten Metallseilen oder Schienen, die entlang von Bojenketten verlaufen, angebracht werden. Dabei werden die Photobioreaktorsegmente parallel zueinander befestigt.

Die Befestigung kann auch über Nähte am Photobioreaktor erfolgen, beispielsweise in dem durch mehrere verstärkte Nähte ein Loch im Folienmaterial ausgebildet und ein Karabinerhaken befestigt wird. Auch kann die Aufhängung über eine speziell dafür vorgesehene und abgetrennte Kammer des Photobioreaktor erfolgen.

Der Photobioreaktor und/oder die Photobioreaktorsegmente und/oder die gesamte Photobioreaktorfarm können vertikal und/oder horizontal aufgehängt werden. In einer derart ausgestalteten Photobioreaktorfarm von je 1.000 m² bis 10.000 m² Fläche wird eine entsprechende Algenproduktion in Gewässern ermöglicht. Die Photobioreaktorfarm kann insbesondere auch eine Fläche von mehreren Quadratkilometern Fläche, insbesondere 10km² bis 100km², einnehmen.

In Photobioreaktorfarmen mit Parallelsetzung von mehreren Photobioreaktoren ist das Öffnen der Sollbruchstellen bei hoher Strömungsbelastung besonders wichtig, da dadurch verhindert wird, dass die Photobioreaktoren gegeneinander treiben und sich dadurch gegenseitig abschatten.

Zudem kann durch die Ausrichtung der Sollbruchstellen erreicht werden, dass sich die einzelnen Photobioreaktoren bei Strömungsbelastung gleichartig verformen und/oder ausrichten.

Um bei langen Photobioreaktorfarmen die Ausbildung von Strömungskanälen durch die geöffneten Sollbruchstellen zu vermeiden, können die Sollbruchstellen auch versetzt auf den einzelnen Photobioreaktoren angeordnet sein.

Insbesondere bei der Photobioreaktorfarm ist eine optimierte Ausrichtung und/oder Verankerung der gesamten Farm vorteilhaft.

Durch diese Photobioreaktorfarm ist eine Skalierung im großindustriellen Produktionsmaßstab möglich.

Im Weiteren wird die Erfindung anhand von Ausführungsbeispielen näher erläutert. Es zeigen
- Figur 1: eine stark schematische Schnittdarstellung eines Photobioreaktors mit Sollbruchstellen bei geringer Strömungsbelastung und
- Figur 2: eine stark schematische Schnittdarstellung eines Photobioreaktors mit Sollbruchstellen bei Überschreitung der Grenzströmung.

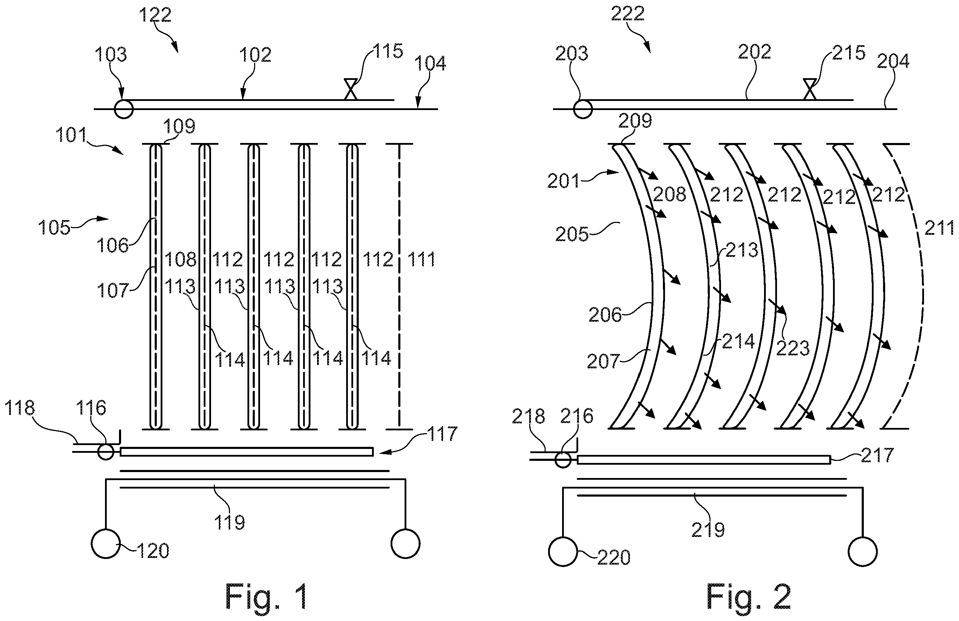

Ein Photobioreaktor 101 weist sechs Kultivierungskammern 105, 108 und 112 auf und ist aus Endlos-Polyethylenfolie 102 gefertigt. Die äußeren seitlichen Begrenzungen des Photobioreaktors 101 sind in Figur 1 nicht gezeigt. Zwischen der ersten und zweiten Kultivierungskammer 105 und 108 ist eine erste Verbindung 106 angeordnet, welche eine Perforationsnaht 107 aufweist. Weitere Kultivierungskammern 112 sind über weitere Verbindungen 113 jeweils mit der vorhergehenden Kultivierungskammer verbunden.

Die Kultivierungskammern 105, 108 und 112 haben jeweils eine Breite von 20,0cm. Die weiteren Verbindungen 113 weisen ebenso weitere Perforationsnähte 114 auf. Die Perforationsnaht 107 und die Perforationsnähte 114 haben eine maximale Sollbruchausdehnung 111. Die Perforationsnaht 107 und die Perforationsnähte 114 sind durch Begrenzungsnähte 109, die jeweils am oberen und unteren Ende der Sollbruchausdehnung 111 sitzen, in ihrer Ausdehnung begrenzt.

Der Photobioreaktor 101 ist an einer oberen Öffnung 103 mit einem Befestigungsseil 104 verbunden und wird über dem Befestigungsseil 104 im Meer 400m vor der Küste gehaltert. Oben am Photobioreaktor 101 befindet sich ein Mehrwegeventil 115.

Über eine untere Öffnung 116 ist ein poröser Versorgungsschlauch 117 innerhalb des Photobioreaktor 101 angeordnet, welcher mit Umgebungsluft begast wird. Des Weiteren ist ein Ernteschlauch 118 durch die untere Öffnung 116 in den Photobioreaktor 101 geführt. Unter dem porösen Versorgungsschlauch 117 befindet sich eine Ballastkammer 119, in der ein Ballastgewicht 120 angeordnet ist.

Die Hauptwasserströmung 122 strömt von hinten gegen den Photobioreaktor 101. Vorliegend sei ein windstiller Tag gegeben. Es liegt keine hohe Scherkraft der Wasserströmung vor und die Perforationsnähte 107 und 114 zwischen den Kultivierungskammern 105, 108 und 112 sind geschlossen.

Durch die Einstellung der Begasung über den porösen Versorgungsschlauch 117, des Mehrwegeventils 115 sowie durch das Ballastgewicht 120 wird der Photobioreaktor 101 im Meer 10cm unterhalb der Wasseroberfläche in Schwebe gehalten.

Vorliegend soll nun ein Sturmereignis gegeben sein, sodass in Hauptströmungsrichtung 222 die Grenzströmung überschritten ist. Dadurch öffnen sich die Perforationsnaht 207 und die weiteren Perforationsnähte 214, sodass das Meerwasser durch die Perforationsnaht 207 und die weiteren Perforationsnähte 214 und um die Kultivierungskammern 205, 208 und 212 herum strömt, wodurch der Strömungswiderstand des Photobioreaktors 201 vermindert ist. Die Wasserströmung 223 tritt gleichmäßig durch alle Perforationsnähte 207 und 214 durch, sodass der Photobioreaktor 201 gleichmäßig über die gesamte Höhe der Sollbruchausdehnung 211 durchströmt wird. Gleichzeitig verformen sich die Kultivierungskammer 205, 208 und 212 gleichmäßig in einer leichten Biegung in Richtung der Hauptwasserströmung 222.

Bei Erreichen des Sturmmaximums mit noch stärkerer Hauptströmung 223 ist zusätzlich das Mehrwegeventil 215 weiter geöffnet, sodass der Photobioreaktor 201 stärker entgast ist und sich folglich der Auftrieb des Photobioreaktors 201 vermindert. Dadurch wird der Photobioreaktor 201 2,5m unterhalb der Wasseroberfläche positioniert und weniger der Wellenbewegung und Strömungsbelastung ausgesetzt.

### Bezugszeichenliste

- 101: Photobioreaktor
- 102: Polyethylenfolie
- 103: obere Öffnung
- 104: Befestigungsseil
- 105: erste Kultivierungskammmer
- 106: erste Verbindung
- 107: Perforationsnaht
- 108: zweite Kultivierungskammer
- 109: Begrenzungsnaht
- 111: Sollbruchausdehnung
- 112: weitere Kultivierungskammer
- 113: weitere Verbindung
- 114: weitere Perforationsnaht
- 115: Mehrwegeventil
- 116: untere Öffnung
- 117: poröser Versorgungsschlauch
- 118: Ernteschlauch
- 119: Ballastkammer
- 120: Ballastgewicht
- 122: Hauptwasserströmung
- 201: Photobioreaktor
- 202: Polyethylenfolie
- 203: obere Öffnung
- 204: Befestigungsseil
- 205: erste Kultivierungskammmer
- 206: erste Verbindung
- 207: Perforationsnaht
- 208: zweite Kultivierungskammer
- 209: Begrenzungsnaht
- 211: Sollbruchausdehnung
- 212: weitere Kultivierungskammer
- 213: weitere Verbindung
- 214: weitere Perforationsnaht
- 215: Mehrwegeventil
- 216: untere Öffnung
- 217: poröser Versorgungsschlauch
- 218: Ernteschlauch
- 219: Ballastkammer
- 220: Ballastgewicht
- 222: Hauptwasserströmung
- 223: Wasserströmung durch Perforationsnähte

## Patentansprüche

1. Photobioreaktor (101, 201) zum Kultivieren von Algen, welcher wenigstens zwei Kultivierungskammern (105, 108, 205, 208) aufweist, welche durch eine erste Verbindung (106, 206) miteinander verbunden sind, **dadurch gekennzeichnet, dass** die erste Verbindung eine Sollbruchstelle (107, 207) aufweist, welche sich bei einer Grenzströmung (222) öffnet, sodass ein strömendes Medium, insbesondere Meerwasser, durch die geöffnete Sollbruchstelle hindurchfließt (223) und somit ein Strömungswiderstand des Photobioreaktors vermindert wird.

2. Photobioreaktor nach Anspruch 1, **dadurch gekennzeichnet, dass** die Sollbruchstelle durch eine Begrenzungsstruktur (109, 209), insbesondere eine verstärkte Begrenzungsnaht, in einer Sollbruchausdehnung begrenzt ist.

3. Photobioreaktor nach Anspruch 1 und 2, **dadurch gekennzeichnet, dass** die Sollbruchstelle eine Sollbruchnaht ist.

4. Photobioreaktor nach Anspruch 1 und 2, **dadurch gekennzeichnet, dass** die Sollbruchstelle ein Wiederverschluss, insbesondere ein Klettverschluss, ist.

5. Photobioreaktor nach einem der vorherigen Ansprüche, **dadurch gekennzeichnet, dass** die Sollbruchstelle eine maximale Sollbruchausdehnung (111, 211) von 5% bis 95% einer vertikalen und/oder horizontalen und/oder schrägen äußeren Abmessung des Photobioreaktors aufweist.

6. Photobioreaktor nach einem der vorherigen Ansprüche, **dadurch gekennzeichnet, dass** der Photobioreaktor weitere Kultivierungskammern (112, 212) aufweist, welche jeweils mit einer weiteren Verbindung (113, 213) an eine angrenzende Kultivierungskammer angeordnet sind.

7. Photobioreaktor nach Anspruch 6, **dadurch gekennzeichnet, dass** die weiteren Verbindungen weitere Sollbruchstellen (114, 214) aufweisen.

8. Photobioreaktor nach einem der vorherigen Ansprüche, **dadurch gekennzeichnet, dass** eine der Sollbruchstellen oder mehrere Sollbruchstellen vertikal und/oder horizontal und/oder schräg, insbesondere parallel zueinander, ausgestaltet sind.

9. Photobioreaktor nach einem der vorherigen Ansprüche, **dadurch gekennzeichnet, dass** die Kultivierungskammern ein lichtdurchlässiges Material (102, 202), insbesondere ein Folienmaterial aus Polyethylen, aufweisen.

10. Photobioreaktor nach einem der vorherigen Ansprüche, **dadurch gekennzeichnet, dass** die Kultivierungskammern Meerwasser als Kultivierungsmedium und/oder wenigstens ein Mehrwegeventil (115, 215) zum Gasaustausch mit dem umgebenden Medium, insbesondere Luft oder Meerwasser, und/oder einen Versorgungsschlauch (117, 217), welcher derart ausgestaltet ist, dass die Kultivierungskammern mit Nährstoffen, insbesondere Gasen und/oder angereichertem Wasser, versorgt werden, aufweisen.

11. Photobioreaktor nach einem der vorherigen Ansprüche, **gekennzeichnet durch** ein Beschwerungselement (120, 220) und/oder ein Verankerungselement, wodurch dem Photobioreaktor eine Ausrichtungsrichtung aufprägbar ist.

12. Photobioreaktor nach einem der vorherigen Ansprüche, **dadurch gekennzeichnet, dass** der Photobioreaktor derart einrichtbar ist, dass durch ein Ent- und/oder Begasen und/oder Verdünnen und/oder Aufkonzentrieren eines in den Kultivierungskammern befindlichen Kultivierungsmediums ein Auftrieb des Photobioreaktors einstellbar ist.

13. Photobioreaktorfarm, welche einen Photobioreaktor nach einem der vorherigen Ansprüche aufweist.

## Claims

1. A photobioreactor (101, 201) for cultivating algae, having at least two cultivation chambers (105, 108, 205, 208), which are connected to one another by a first connection (106, 206), **characterized in that** the first connection has a predetermined breaking point (107, 207), which opens in the event of a threshold current (222), so that a flowing medium, in particular seawater, flows through the opened predetermined breaking point (223), thus reducing a current resistance of the photobioreactor.

2. The photobioreactor according to claim 1, **characterized in that** the extent of the predetermined rupture at the predetermined breaking point is limited by a limiting structure (109, 209), in particular a reinforced limiting seam.

3. The photobioreactor according to claims 1 and 2, **characterized in that** the predetermined breaking point is a predetermined breaking seam.

4. The photobioreactor according to claims 1 and 2, **characterized in that** the predetermined breaking point is a reclosure system, in particular a hook-and-loop fastener.

5. The photobioreactor according to one of the afore-mentioned claims, **characterized in that** the predetermined breaking point has a maximum predetermined rupture extension (111, 211) of 5% to 95% of a vertical and/or horizontal and/or diagonal outer dimension of the photobioreactor.

6. The photobioreactor according to one of the afore-mentioned claims, **characterized in that** the photobioreactor comprises additional cultivation chambers (112, 212), which can be disposed at an adjacent cultivation chamber using a respective additional connection (113, 213).

7. The photobioreactor according to claim 6, **characterized in that** the additional connections comprise additional predetermined breaking points (114,214).

8. The photobioreactor according to one of the afore-mentioned claims, **characterized in that** one of the predetermined breaking points or several predetermined breaking points are formed vertically and/or horizontally and/or diagonally, in particular parallel to one another.

9. The photobioreactor according to one of the afore-mentioned claims, **characterized in that** the cultivation chambers have a translucent material (102, 202), in particular a foil material made of polyethylene.

10. The photobioreactor according to one of the afore-mentioned claims, **characterized in that** the cultivation chambers comprise seawater as a cultivation medium and/or at least one multi-way valve (115, 215) for gas exchange with the surrounding medium, in particular air or seawater, and/or a supply tube (117, 217), which is designed in such a manner that the cultivation chambers can be supplied with nutrients, in particular gases and/or enriched water.

11. The photobioreactor according to one of the afore-mentioned claims, **characterized by** a ballast element (120, 220) and/or an anchoring element with which the photobioreactor can be oriented.

12. The photobioreactor according to one of the afore-mentioned claims, **characterized in that** the photobioreactor can be arranged in such a manner that by introducing and/or evacuating gas, and/or diluting and/or concentrating a cultivation medium contained in the cultivation chambers, a buoyancy of the photobioreactor can be adjusted.

13. A photobioreactor farm, which comprises a photobioreactor according to one of the afore-mentioned claims.

## Revendications

1. Photobioréacteur (101, 201) servant à cultiver des algues qui comporte au moins deux chambres de culture (105, 108, 205, 208) reliées entre elles par une première liaison (106, 206), **caractérisé en ce que** la première liaison comporte un point de rupture (107, 207) qui s'ouvre lorsqu'un écoulement limite (222) est atteint, de sorte qu'un milieu en écoulement, en particulier de l'eau de mer, s'écoule (223) à travers le point de rupture ouvert et une résistance à l'écoulement du photobioréacteur est réduite.

2. Photobioréacteur selon la revendication 1, **caractérisé en ce que** l'élargissement du point de rupture est limité par une structure de limitation (109, 209), en particulier un joint de limitation renforcé.

3. Photobioréacteur selon la revendication 1 et 2, **caractérisé en ce que** le point de rupture est un joint de rupture.

4. Photobioréacteur selon la revendication 1 et 2, **caractérisé en ce que** le point de rupture est refermable, en particulier une fermeture à crochets et boucles.

5. Photobioréacteur selon l'une des revendications précédentes, **caractérisé en ce que** le point de rupture présente un élargissement de point de rupture maximal (111, 211) compris entre 5% et 95% d'une dimension extérieure verticale et/ou horizontale et/ou oblique du photobioréacteur.

6. Photobioréacteur selon l'une des revendications précédentes, **caractérisé en ce que** le photobioréacteur comporte des chambres de culture supplémentaires (112, 212), qui sont disposées avec une liaison supplémentaire (113, 213) respective au niveau d'une chambre de culture voisine.

7. Photobioréacteur selon la revendication 6, **caractérisé en ce que** les liaisons supplémentaires comportent des points de rupture supplémentaires (114,214).

8. Photobioréacteur selon l'une des revendications précédentes, **caractérisé en ce que** l'un des points de rupture ou plusieurs points de rupture sont formés verticalement et/ou horizontalement et/ou obliquement, en particulier parallèlement les uns aux autres.

9. Photobioréacteur selon l'une des revendications précédentes, **caractérisé en ce que** les chambres de culture comportent un matériau translucide (102, 202), en particulier un matériau de feuille en polyéthylène.

10. Photobioréacteur selon l'une des revendications précédentes, **caractérisé en ce que** les chambres de culture comportent de l'eau de mer en tant que milieu de culture et/ou au moins une soupape à voies multiples (115, 215) pour permettre un échange gazeux avec le milieu environnant, en particulier de l'air ou de l'eau de mer, et/ou un tuyau d'alimentation (117, 217) formé de telle manière que les chambres de culture sont alimentées en nutriments, en particulier en gaz et/ou en eau enrichie.

11. Photobioréacteur selon l'une des revendications précédentes, **caractérisé par** un élément de lest (120, 220) et/ou un élément d'ancrage au moyen duquel le photobioréacteur peut être orienté.

12. Photobioréacteur selon l'une des revendications précédentes, **caractérisé en ce que** le photobioréacteur peut être aménagé de telle manière qu'une flottabilité du photobioréacteur peut être ajustée par dégazage et/ou gazage et/ou dilution et/ou concentration d'un milieu de culture se trouvant dans les chambres de culture.

13. Parc de photobioréacteurs comprenant un photobioréacteur selon l'une des revendications précédentes.
